# EUROPÄISCHE PATENTSCHRIFT

(11) **EP 0 938 872 B1**
(45) Veröffentlichungstag und Bekanntmachung des Hinweises auf die Patenterteilung: **03.07.2002**
(21) Anmeldenummer: 98810957.5
(22) Anmeldetag: 24.09.1998
(51) Int. Cl.: A61B 17/70

(54) **Schwenkbares Befestigungssystem an einer Knochenschraube**
Pivotable attachment system for a bone screw
Système de fixation articulée pour une vis à os

(30) Priorität: 02.02.1998 EP 98810076
(43) Veröffentlichungstag der Anmeldung: 01.09.1999
(73) Patentinhaber: Sulzer Orthopädie AG, 6340 Baar (CH)
(72) Erfinder: Studer, Armin, 6312 Steinhausen (CH); Donno, Cosimo, 8400 Winterthur (CH); Fröhlich, Markus, 8362 Balterswil (CH)
(74) Vertreter: Manitz, Finsterwald & Partner Gbr

(56) Entgegenhaltungen:
- EP-A- 0 504 103
- EP-A- 0 614 649
- WO-A-96/29947
- DE-A- 19 542 116

## Beschreibung

Die Erfindung handelt von einem schwenkbaren Befestigungssystem zwischen einer Knochenschraube mit einem kugelförmigen Kopfteil und einem Aufnahmeteil, welches am Ende einer Durchgangsbohrung das Kopfteil mit einer Schulter an dessen Unterseite umfasst und welches eine Klemmschraube in der Durchgangsbohrung aufnimmt, mit der das Kopfteil in einer wählbaren Schwenkstellung gegen die Schulter anpressbar ist.

In der EP-A-0 614 649 wird eine Knochenschraube gezeigt, die vor dem Eindrehen in ein Implantat durch ein Aufnahmeteil gesteckt werden muss, welches beim Eindrehen hinderlich ist. Gerade bei Wirbelkorrekturen werden mehrere solcher Knochenschrauben, die in verschiedenen Wirbeln eingeschraubt sind, über Verbindungsstangen und Aufnahmeteile miteinander verbunden. Das Aufnahmeteil ist turmartig ausgeführt und nimmt den Kugelkopf der Knochenschraube in sich auf. Die Knochenschraube ist an einer Gegenschale mit ihrem Kugelkopf schwenkbar gelagert und kann entweder direkt durch eine Klemmschraube oder indirekt über Zwischenglieder wie Verbindungsstange und Druckscheibe durch eine Klemmschraube auf das Aufnahmeteil festgesetzt werden.

Ähnliche Überlegungen zeigt die DE-A-195 42 116 für ein plattenförmiges Aufnahmeteil, welches vor dem Festsetzen von Kugelflächen des Schraubenkopfes, um den gemeinsamen Mittelpunkt dieser Kugelflächen schwenkbar ist.

Beiden Anordnungen ist gemeinsam, dass die Knochenschraube durch das Aufnahmeteil hindurch eingeschraubt werden muss.

Aufgabe der Erfindung ist es, diesem Umstand abzuhelfen. Diese Aufgabe wird dadurch gelöst, dass das kugelförmige Kopfteil als ein separates Teil ausgeführt ist, welches mit der Knochenschraube verschraubbar ist, um das Aufnahmeteil nach dem Implantieren der Knochenschraube aufsetzen und mit dieser verbinden zu können.

Die Anordnung hat den Vorteil, dass die Knochenschraube beispielsweise als Pedikelschraube beim Einschrauben frei zugänglich ist. Ein Operateur kann während dem Eindrehen Lage und Sitz der Schraube, sowie den Zustand vom Knochengewebe überprüfen. Bei der Verbindung von mehreren Pedikelschrauben können die Aufnahmeteile provisorisch auf vorgebogenen Verbindungsstangen befestigt und durch Aufsetzen an den Pedikelschrauben in ihrer Lage korrigiert werden, um anschliessend separate Kugelköpfe auf den Pedikelschrauben zu befestigen und Klemmschrauben lose einzudrehen. Die provisorisch festgesetzten Verbindungsstangen können noch einmal in ihrer Befestigung zum Aufnahmeteil gelockert werden und anschliessend können alle Verbindungen gleichmässig angezogen werden. Dieses Vorgehen empfiehlt sich, wenn die Wirbel zueinander ausgerichtet sind. Wenn jedoch Verschiebungen der Wirbel zueinander notwendig sind, wird auf eine Lockerung der auf Verbindungsstangen ausgerichteten Aufnahmeteile bewusst verzichtet. Ein weiterer Vorteil ist eine flache Bauweise vom Aufnahmeteil und die Möglichkeit, bestehende Standard-Pedikelschrauben zu verwenden, um zu einem vernünftigen Schwenkbereich und zu einem erleichterten Einsetzen der Pedikelschrauben zu kommen.

Vorteilhafte Weiterbildungen der Erfindung sind in den Ansprüchen 2 bis 13 aufgezeigt.

Die Ausführung des separaten kugelförmigen Kopfteils als Schraubenmutter, die mit einem schraubenförmigen Fortsatz der Knochenschraube verschraubbar ist erweitert den Anwendungsbereich bei Standard-Pedikelschrauben. Dadurch, dass diese Schraubenmutter durch Schlitze geschwächt wird, setzt sie sich beim Zusammenpressen auf ihrem Gegengewinde fest. Die Knochenschraube muss daher nicht notwendigerweise einen Anschlag auf ihrem schraubenförmigen Fortsatz für die kugelförmige Schraubenmutter aufweisen. Die Schraubenmutter kann daher auch in ihrer Höhe verstellt und anschliessend durch Zusammenpressen auf dem Gegengewinde festgesetzt werden. Eingriffsflächen in Form von Einbuchtungen erleichtern die Höhenverstellung der kugelförmigen Schraubenmutter. Die Schulter des Aufnahmeteils und die Anpressfläche der Klemmschraube sind als konische Flächen ausgebildet, deren halber Konuswinkel α in bestimmten Grenzen 8° < α < 25° liegen sollte, um eine gute Klemmwirkung und geringen Platzbedarf zu erreichen. Mit den konischen Flächen sind gegenüber einer Kugelfläche die Kraftangriffspunkte auch bei elastischen und leicht plastischen Deformationen praktisch noch am gleichen Ort und kaum von Herstelltoleranzen abhängig. Die Einfederung der durch Schlitze geschwächten Schraubenmutter kann auf diese konstanten Kraftangriffspunkte abgestimmt werden. Zusätzlich zur Reibung zwischen konischen Flächen und Kugelfläche findet auch eine elastische und eine plastische Deformation in Form einer Abplattung statt, die Material im Bereich der Einbuchtungen der Kugelfläche vorstehen lässt und einer Drehung ebenfalls entgegenwirkt. Diese Art der Verbindung gestattet es Knochenschrauben mit und ohne Kragen zu verwenden, Höhenverstellungen am schraubenförmigen Fortsatz der Knochenschraube vorzunehmen und an der eigentlichen Knochenschraube Gewindedurchmesser zu verwenden, die grösser als der Innendurchmesser der Schulter im Aufnahmeteil sind.

Wenn die kugelförmige Schraubenmutter nur von oben her Schlitze aufweist und auf ihrer unteren Seite ein Hals angeformt ist, der als geschlossener Ring ausserhalb des Aussendurchmessers des schraubenförmigen Fortsatzes auf einer Schulter der Knochenschraube abgestützt ist, lässt sich der geschlossene Ring beim Eindrehen der Schraubenmutter gegen die Schulter der Knochenschraube verspannen. Dies hat den Vorteil, dass bei einem bestimmten an der kugelförmigen Schraubenmutter angreifenden Übertragungsmoment geringere Biegespannungsspitzen an dem schraubenförmigen Fortsatz der Knochenschraube auftreten.

Eine weitere Verbesserung besteht darin, die Klemmschraube auf ihrer Klemmfläche mit einem Gewinde in Form einer spiralförmig angeordneten Rille zu versehen, wobei diese Rille respektiv die Gewindezahnung gleichgerichtet wie das Aussengewinde der Klemmschraube ist und eine gleiche Steigung wie das Aussengewinde aufweist. Mit einer solchen Massnahme ist sichergestellt, dass die Richtung der spiralförmigen Rille und ihre Relativbewegung gegenüber dem kugelförmigen Kopfteil beim Klemmen zusammenfallen. Der vorstehende Gewindeteil kann sich und Kugelfläche begrenzt plastisch deformieren, sodass eine kraftschlüssige und formschlüssige Verbindung entsteht, die zur Übertragung von Biegemomenten auf einen kugelförmigen Kopf besonders geeignet ist. Grundsätzlich lässt sich mit dieser Massnahme jeder Kugelkopf, der in einer Gewindebohrung gefangen ist und mit einer Klemmschraube mit innenliegender Anpressfläche festgesetzt wird, in der Übertragung eines maxiamlen Biegemomentes verbessern, wenn die Anpressfläche ein spiralförmiges Gewinde aufweist, welches gleichgerichtet wie das Aussengewinde der Klemmschraube ist und welches die gleiche Steigung aufweist. Da die Tiefe des spiralförmigen Gewindes zum Klemmen nicht gross sein muss, hat es sich bewährt, das spiralförmige Gewinde mehrgängig auszuführen. Eine Verbesserung der übertragbaren Momente tritt auch ein, wenn die Deformationen nur im elastischen Bereich sind.

Im folgenden wird die Erfindung anhand von Ausführungsbeispielen beschrieben. Es zeigen:
- Fig. 1: Schematisch eine Pedikelschraube mit einem als Schraube ausgebildeten separaten kugelförmigen Kopfteil;
- Fig. 2: schematisch eine Pedikelschraube mit einem als Mutter ausgebildeten separaten kugelförmigen Kopfteil;
- Fig. 3: schematisch eine Standard-Pedikelschraube mit einem als Mutter ausgebildeten separaten kugelförmigen Kopfteil und mit einem verschwenkbaren Aufnahmeteil;
- Fig. 4: schematisch eine Anordnung nach Figur 3 mit fixierter Verbindungsstange;
- Fig. 5: schematisch eine kragenlose Pedikelschraube mit einem in der Höhe verstellbaren kugelförmigen Kopfteil, welches als Mutter ausgebildet ist;
- Fig. 6: schematisch eine Anordnung nach Figur 3, bei der für eine nicht aus der Achse schwenkbare Befestigung Klemmschraube und kugelförmige Mutter durch eine anders dimensionierte Befestigungsmutter ersetzt wurden;
- Fig. 7 bis 14: schematisch jeweils eine Seitenansicht einer mit Schlitz und Eingriffsfläche versehenen kugelförmigen Schraubenmutter und deren Projektionen in Richtung der Schraubenachse;
- Fig. 15: schematisch einen vergrösserten Ausschnitt von Figur 3, aus dem die halben Konuswinkel an Aufnahmeteil und Klemmschraube ersichtlich sind;
- Fig. 16: schematisch den Kopf eines Schraubwerkzeuges, welches für Schraubenmuttern der Figuren 7, 8 und 9 einsetzbar ist;
- Fig. 17: schematisch in kleinerem Massstab eine Ansicht eines Aufnahmeteils, das mit einer Knochenschraube verbunden ist;
- Fig. 18: schematisch und vergrössert im Längsschnitt eine weitere Ausführung einer kugelförmigen Schraubenmutter mit einem angeformten Hals zur Abstützung auf einer Schulter der Knochenschraube;
- Fig. 19: schematisch die Schraubenmutter von Fig. 18 in einer Draufsicht;
- Fig. 20: schematisch und vergrössert im Längsschnitt eine weitere Ausführung einer Klemmschraube;
- Fig. 21: schematisch und vergrössert eine Draufsicht der Klemmschraube von Fig. 20; und
- Fig. 22: schematisch einen nochmals vergrösserten Ausschnitt der Klemmschraube von Fig. 20 mit einem Innengewinde auf der Anpressfläche für das kugelförmige Kopfteil.

In den Figuren ist ein schwenkbares Befestigungssystem zwischen einer Knochenschraube 1 mit einem kugelförmigen Kopfteil 2 und einem Aufnahmeteil 3 gezeigt. Das Aufnahmeteil besitzt eine Durchgangsbohrung 4 mit einer Schulter am Ende, die das Kopfteil 2 auf seiner Unterseite umfasst und einen Anschlag bildet, wenn das Kopfteil 2 in einer einstellbaren Schwenkstellung 8 durch eine Klemmschraube 7 angepresst wird. Das Kopfteil 2 ist als separates Teil 9 ausgeführt, welches mit der Knochenschraube 1 verschraubbar ist, um das Aufnahmeteil 3 nach dem Implantieren der Knochenschraube 1 aufsetzen und mit dieser verbinden zu können.

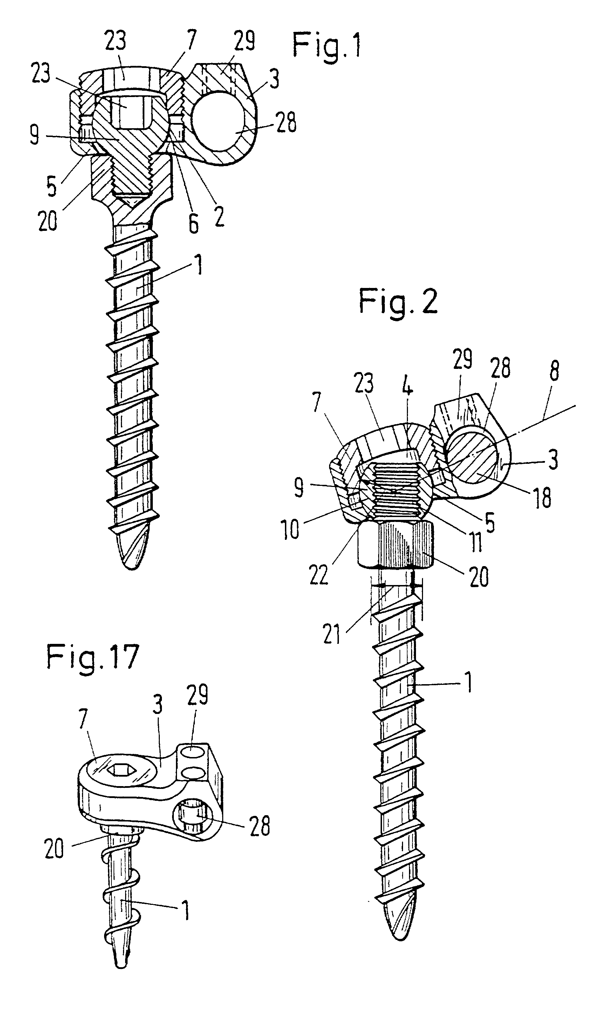

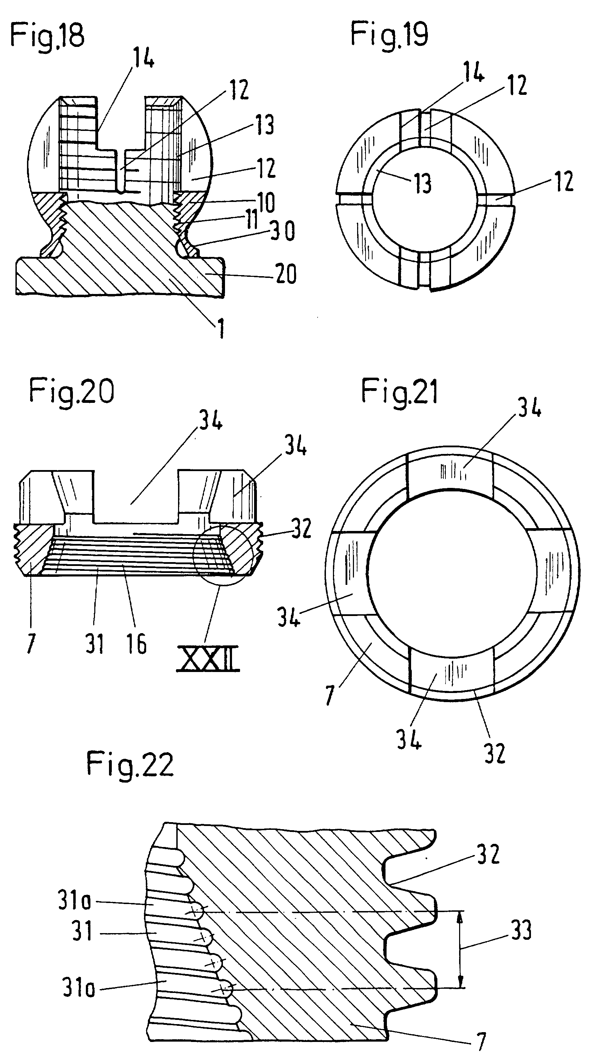

In den Figuren 1, 2, 3, 4, 5, 15 und 17 ist immer wieder ein gleiches Aufnahmeteil 3 gezeigt, das als Befestigungssystem 17 in einer Bohrung 28 eine Verbindungsstange 18 aufnimmt und an dieser mit einer oder zwei Madenschrauben 24 befestigt ist, welche in Gewindebohrungen 29 versenkt sind. Seitlich angeformt ist eine Lasche (Figur 17) mit einer Durchgangsbohrung 4, die quer und versetzt zur Bohrung 28 angeordnet ist und am unteren Ende eine nach innen vorstehende ringförmige Schulter 5 besitzt. Über der Schulter 5 ist die Durchgangsbohrung 4 soweit verbreitert, dass ein kugelförmiger Kopfteil 9, 10 einer Pedikelschraube 1 durch die Schulter 5 gefangen ist und mit einer Klemmschraube 7 an dieser anpressbar ist. Da die Pedikelschrauben 1 mit einem kugelförmigen Kopfteil 2 ausgestattet sind, welches als separates Teil 9 mit der Pedikelschraube 1 verschraubbar ist, bestehen viel mehr Freiheiten in der Formgebung der einzelnen Pedikelschrauben ohne dass auf eine Schwenkbarkeit um einen Kugelkopf verzichtet werden muss.

In Figur 1 ist die Pedikelschraube 1 mit einem Kragen 20 versehen der einen Aussensechskant aufweist, um die Schraube 1 einzudrehen. Der Kragen 20 besitzt eine Bohrung mit Innengewinde, um nach dem Aufsetzen des Aufnahmeteils 3 das separate kugelförmige Kopfteil 9 an einem Innensechskant 23 aufzunehmen und in diese Bohrung einzuschrauben. Das kugelförmige Kopfteil 9 wird gegen einen Anschlag festgezogen der durch ein auslaufendes Gewinde oder die Stirnfläche des Kragens 20 gegeben ist. Wesentlich ist, dass die Schulter 5 mit soviel Spiel durch die Unterseite 6 des Kugelkopfes 2 gefangen ist, dass der vorgesehene Schwenkbereich beim Anliegen der Schulter 5 am Kugelkopf 2 durchfahren werden kann. Die Klemmschraube 7 ist in der Darstellung lose eingeschraubt und sitzt mit einer konischen Anpressfläche auf dem Kugelkopf 2 auf. Die Klemmschraube 7 ist als Deckelschraube mit einem Innensechskant 23 ausgeführt. Statt des Innensechskant 23 könnten auch zwei aus der Achse verschobene Sacklöcher als Angriffsflächen zum Anziehen der Deckelschraube 7 dienen und annähernd geschlossene Räume zwischen Deckelschraube 7 und Schulter 5 schaffen.

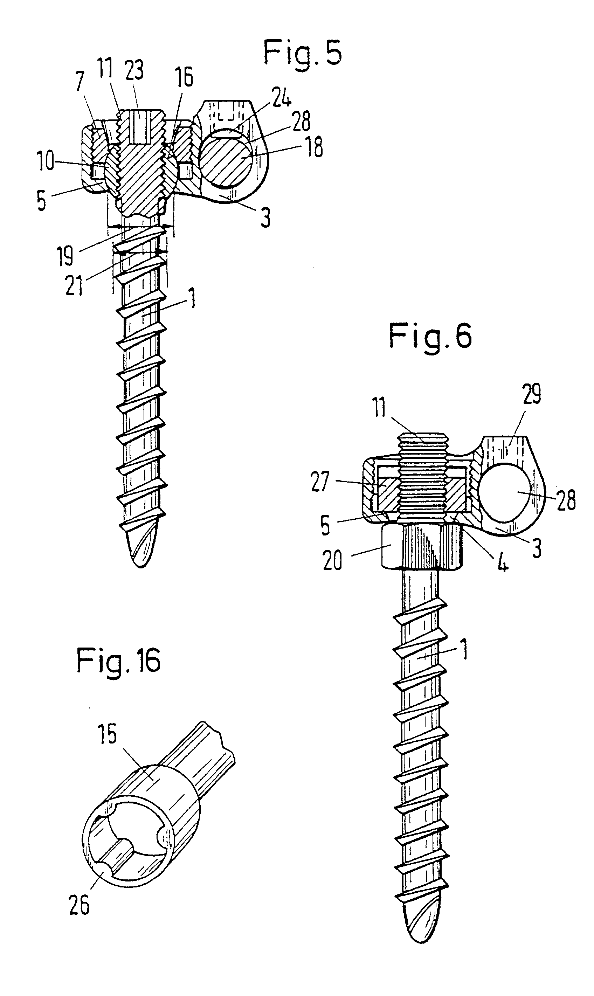

In Figur 2 ist ein gleiches Aufnahmeteil 3 mit einer gleichen Deckelschraube 7 in einer Schwenkstellung 8 auf einem separaten kugelförmigen Kopfteil 9 befestigt, welches als Schraubenmutter 10 ausgeführt ist, die fest mit einem schraubenförmigen Fortsatz 11 der Pedikelschraube 1 verschraubt ist und gegen deren Kragen 20 aufliegt. Das Gewinde der eigentlichen Pedikelschraube 1 hat einen Durchmesser 21, der ohne weiteres grösser als der Innendurchmesser 19 (Figur 5) der Schulter 5 sein darf. Der Kugelradius 22 der Schraubenmutter 10 steht in einer bestimmten Relation zu den Durchmessern der Anpressflächen von Schulter 5 und Klemmschraube 7, die durch die Konuswinkel von mittleren konischen Anpressflächen gegeben ist, wie später gezeigt wird.

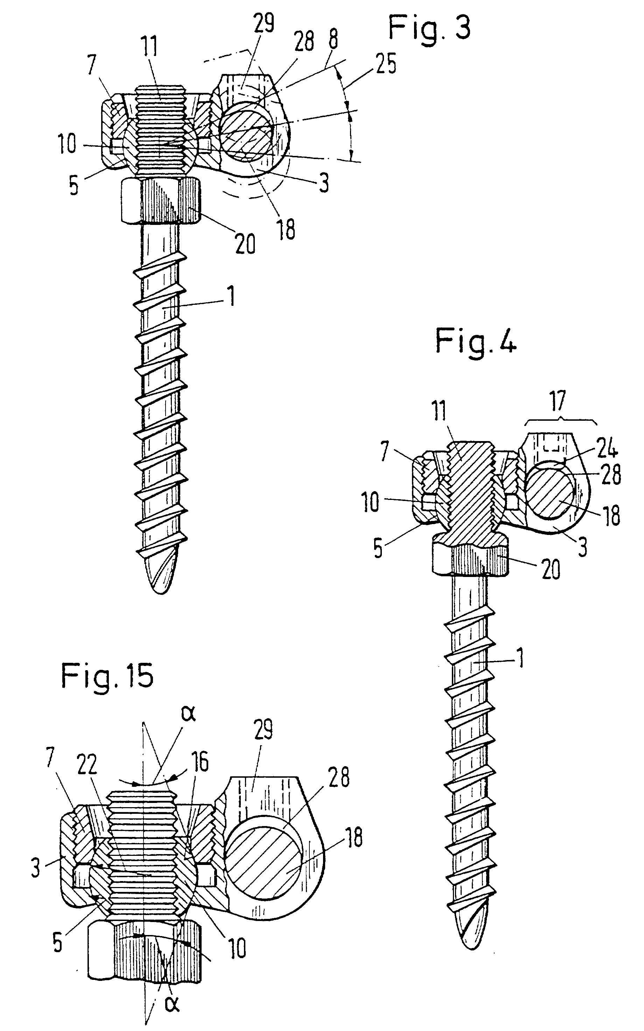

In den Figuren 3, 4 und 15 ist eine Standard-Pedikelschraube 1 mit einem relativ langen schraubenförmigen Fortsatz 11 gezeigt. In Figur 3 ist die Klemmschraube 7 nur leicht angezogen und erlaubt aus einer mittleren Schwenkstellung Abweichungen 25 der Schwenkstellung 8 für das Aufnahmeteil, die in der Grössenordnung von plus oder minus 15° liegen, wenn, wie in Figur 15 ersichtlich ist, die Anpressfläche der Stützschulter 5 und die Anpressfläche 16 der Klemmschraube 7 konische Flächen mit einem halben Konuswinkel 8° < α < 25° sind. Die konischen Anpressflächen haben den Vorteil, dass die Kraftangriffspunkte am Kugelkopf 10 erhalten bleiben, wenn sich dieser aufgrund einer bewussten Schwächung gegen innen deformiert. Ein halber Konuswinkel α ≈ 20° wird für diesen Fall als vorteilhaft angesehen. Da der schraubenförmige Fortsatz 11 der gezeigten Standard-Pedikelschraube 1 länger als die Höhe vom Kugelkopf 10 ausgeführt ist, wird in der Klemmschraube 11 eine konische Bohrung angebracht, welche die maximal vorgesehene Schwenkung zulässt. Die Klemmschraube 10 ist auf ihrer oberen Seite mit Aussparungen versehen, die das Ansetzen eines Eindrehwerkzeuges erlauben. In Figur 4 ist die Klemmschraube 7 auf der kugelförmigen Schraubenmutter 10 festgesetzt. Solange sich die Schraubenmutter 10 nicht soweit nach innen deformiert, dass das Gewindespiel zwischen Schraubenmutter 10 und dem schraubenförmigen Fortsatz 11 aufgehoben wird, sind einzig die durch das Festziehen der Schraubenmutter 10 auf dem Kragen und Gewinde erzeugten Reibungskräfte dafür verantwortlich, welches Drehmoment bezüglich der Schraubenachse vom Aufnahmeteil 3 auf die Pedikelschraube übertragbar ist.

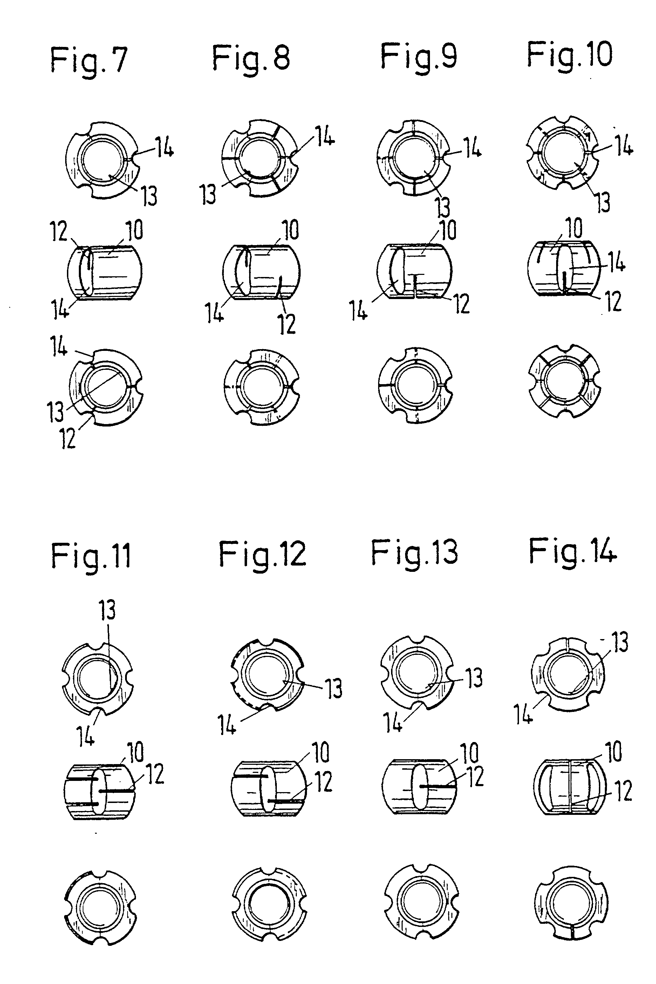

Die Figuren 7 bis 14 zeigen daher eine Vielfalt von künstlich geschwächten kugelförmigen Schraubenmuttern 10, die in diesem Fall durch zusätzliche Schlitze 12 geschwächt sind, welche durch das Gewinde gehen, um beim Zusammenpressen zwischen den konischen Anpressflächen 16, 5 eine Deformation der Gewindegänge nach innen und ein zusätzliches Verklemmen auf dem schraubenförmigen Fortsatz 11 zu erreichen. In den Figuren 7, 8, 9, 10, 14 liegen die Schlitze in Ebenen die durch die Achse der Pedikelschraube gehen. In den Figuren 11, 12, 13 liegen die Schlitze in Ebenen, die senkrecht zur Achse der Pedikelschraube liegen. Andere Schwächungen durch Einstiche und Kerben sind ebenfalls denkbar, wenn sie zu einer Reduktion des Gewindedurchmessers 13 an der kugelförmigen Schraubenmutter 10 führen. An der Aussenseite der Schraubenmutter 10 sind Eingriffsflächen 14 in Form von zylindrischen Ausnehmungen angebracht, die das Eindrehen mit einem Schraubwerkzeug 15 (Figur 16) erlauben, welches passende Vorsprünge 26 aufweist.

In Figur 5 ist eine Verankerung der Schraubenmutter 10 ohne Anschlag gezeigt. Das Eindrehen der Pedikelschraube erfolgt am oberen Ende an einem Innensechskant 23. Nach dem Eindrehen der Pedikelschraube 1, deren Gewindedurchmesser 21 ebenfalls grösser als der Innendurchmesser 19 der Schulter 5 sein darf, kann das Aufnahmeteil 3 aufgesetzt werden. Anschliessend wird die kugelförmige Schraubenmutter 10 auf eine passende Höhe am schraubenförmigen Fortsatz 11 eingeschraubt.
Anschliessend wird die Klemmschraube 7 eingeschraubt und das Aufnahmeteil 3 in eine passende Richtung geschwenkt. Mit dem Festziehen der Klemmschraube 7 gegenüber dem Aufnahmeteil 3 setzt sich die deformierbare kugelförmige Mutter 10 auf dem Gewinde fest und wird blockiert. Mit Hilfe der konischen Anpressflächen 16, 5 und der Anzahl und Grösse der Aussparungen 14 können gezielt lokale plastische Deformationen erzeugt werden, die zwischen Schraubenmutter 10 und den Anpressflächen 16, 5 eine starre Verbindung erzeugen.

Figur 6 zeigt mit reduzierten Elementen ein nicht zur Erfindung gehörendes Beispiel, welches das Verständnis der Erfindung erleichert. Die Standard-Pedikelschraube 1 und das Aufnahmeteil 3 aus Figur 3 sind direkt mit einer Befestigungsmutter 27 in einer Ebene zueinander bezüglich Drehwinkel einstellbar fixiert. Die Klemmung der Schulter 5 findet zwischen dem Kragen 20 und der Befestigungsmutter 27 statt.

Die Schraubenmutter 10 in den Figuren 18 und 19 ist nur etwa auf zwei Drittel von oben mit Schlitzen 12 versehen, sodass im unteren Drittel ein zusammenhängender Gewindeteil für das Innengewinde 13 entsteht. Zusätzlich ist ein Hals 30 angeformt der schräg nach aussen wegsteht und auf einer Schulter 20 der Knochenschraube 1 abgestützt ist. Mit einem nicht gezeigten Eindrehwerkzeug kann die Schraubenmutter 10 an Eingriffsflächen 14 gefasst und unter Vorspannung gegen die Schulter 20 verschraubt werden. Ein an der Kugelfläche der Schraubenmutter angreifendes Biegemoment bewirkt in erster Linie eine Vergrösserung der Druckspannungen im Hals 30 und eine Erhöhung der Zugspannungen im schraubenförmigen Fortsatz 11 der Knochenschraube 1. Im Gegensatz zu einer reinen Biegebelastung mit einer neutralen Phase und mit gegen aussen wachsenden Zug- und Druckspannungen ist der schraubenförmige Fortsatz 11 auf seinem ganzen Querschnitt auf Zug belastet, wobei wesentlich niedrigere Spannungsspitzen auftreten.

In den Figuren 20, 21 und 22 ist eine generelle Verbesserung von Klemmschrauben 7 für Kugelköpfe gezeigt. Die Anpressfläche 16 ist der Ausschnitt von einem Innenkonus, der mit einem Mehrfachgewinde 31, 31a in Form von spiralförmigen Rinnen versehen wurde, wobei eine Rinne 31a gleichgerichtet wie das Aussengewinde 32 der Klemmschraube 7 ist und die Steigung 33 wie das Aussengewinde besitzt, um eine höhere gegenseitige Durchdringung von Kugelfläche und Mehrfachgewinde zu erreichen. Zur Übertragung eines grossen Anzugmomentes sind vier kreuzweise angeordnete Nuten 34 im oberen Bereich der Klemmschraube angebracht.

## Patentansprüche

1. Schwenkbares Befestigungssystem zwischen einer Knochenschraube (1) mit einem kugelförmigen Kopfteil (2) und einem Aufnahmeteil (3), welches am Ende einer Durchgangsbohrung (4) das Kopfteil (2) mit einer Schulter (5) an dessen Unterseite (6) umfasst und welches eine Klemmschraube (7) in der Durchgangsbohrung (4) aufnimmt, mit der das Kopfteil (2) in einer wählbaren Schwenkstellung (8) gegen die Schulter (5) anpressbar ist, **dadurch gekennzeichnet, dass** das kugelförmige Kopfteil (2) als ein separates Teil (9) ausgeführt ist, welches mit der Knochenschraube verschraubbar ist, um das Aufnahmeteil (3) nach dem Implantieren der Knochenschraube (1) aufsetzen und mit dieser verbinden zu können.

2. Befestigungssystem nach Anspruch 1, **dadurch gekennzeichnet, dass** das kugelförmige Kopfteil (2) als eine Schraubenmutter (10) ausgeführt ist, die mit einem schraubenförmigen Fortsatz (11) der Knochenschraube (1) verschraubbar ist.

3. Befestigungssystem nach Anspruch 2, **dadurch gekennzeichnet, dass** die Schraubenmutter (10) durch mindestens einen Schlitz von ihrem Innengewinde (13) gegen aussen geschwächt ist, um das Innengewinde (13) beim Zusammenpressen der kugelförmigen Schraubenmutter (10) durch eine Deformation der Gewindegänge auf dem schraubenförmigen Fortsatz (11) zu fixieren.

4. Befestigungssystem nach Anspruch 2 oder 3, **dadurch gekennzeichnet, dass** die kugelförmige Schraubenmutter (10) Eingriffsflächen (14) aufweist, die das Ansetzen eines Schraubwerkzeuges (15) ermöglichen.

5. Befestigungssystem nach einem der Ansprüche 1 bis 4, **dadurch gekennzeichnet, dass** die Schulter (5) und eine Anpressfläche (16) der Klemmschraube (7) als konische Flächen ausgeführt sind.

6. Befestigungssystem nach Anspruch 5, **dadurch gekennzeichnet, dass** die konischen Flächen einen halben Konuswinkel 8° < α < 25° aufweisen.

7. Befestigungssystem nach Anspruch 6, **dadurch gekennzeichnet, dass** die konischen Flächen einen halben Konuswinkel α ≈ 20° aufweisen.

8. Befestigungssystem nach einem der Ansprüche 1 bis 7, **dadurch gekennzeichnet, dass** das Aufnahmeteil (3) eine weitere Befestigung (17) für eine Verbindungsstange (18) aufweist.

9. Befestigungssystem nach einem der Ansprüche 1 bis 8, **dadurch gekennzeichnet, dass** die Knochenschraube (1) einen über den Innendurchmesser (19) der Schulter (5) nach aussen vorstehenden Kragen (20) aufweist.

10. Befestigungssystem nach einem der Ansprüche 1 bis 9, **dadurch gekennzeichnet, dass** der Gewindedurchmesser (21) des in den Knochen einbringbaren Gewindes der Knochenschraube (1) grösser ist als der Innendurchmesser (19) der Schulter (5).

11. Befestigungssystem nach einem der Ansprüche 2 bis 4, **dadurch gekennzeichnet, dass** die kugelförmige Schraubenmutter (10) einen Hals (30) aufweist, der einen geschlossenen Ring bildet und der sich ausserhalb des Aussendurchmessers des schraubenförmigen Fortsatzes (11) auf einer Schulter (20) der Knochenschraube (1) abstützt.

12. Befestigungssystem nach einem der Ansprüche 1 bis 7, **dadurch gekennzeichnet, dass** die Anpressfläche (16) der Klemmschraube (7) mit einem Innengewinde (31) versehen ist, welches gleichgerichtet wie das Aussengewinde (32) der Klemmschraube (7) ist und eine gleiche Steigung (33) aufweist, damit das Innengewinde (31) beim Anziehen der Klemmschraube (7) auf dem kugelförmigen Kopfteil (2) eine Rinne einformt, welche das am kugelförmigen Kopfteil übertragbare Drehmoment verbessert.

13. Befestigungsschraube nach Anspruch 12, **dadurch gekennzeichnet, dass** das Innengewinde (31) mehrgängig ausgeführt ist, um die Anzahl der am Kopfteil (2) angreifenden Gewindespitzen zu erhöhen.

## Claims

1. Pivotal securing system between a bone screw (1) having a spherical head part (2) and a reception part (3) which, at the end of a passage bore (4), has a shoulder (5) surrounding the head part (2) at its lower side (6) and which receives a clamping screw (7) in the passage bore (4) by means of which the head part (2) can be pressed against the shoulder (5) in a selectable pivotal position (8), **characterised in that** the spherical head part (2) is executed as a separate part (9) which can be screwed to the bone screw in order to be able to mount the reception part (3) after the implantation of the bone screw (1) and to connect it to the latter.

2. Securing system in accordance with claim 1, **characterised in that** the spherical head part (2) is executed as a screw nut (10) which can be screwed onto a screw-shaped extension (11) of the bone screw (1).

3. Securing system in accordance with claim 2 **characterised in that** the screw nut (10) is weakened by at least one slit from its inner thread (13) towards the outside in order to fix the inner thread (13) during the compression of the spherical scrcew nut (10) by a deformation of the thread turns on the screw-shaped extension (11).

4. Securing system in accordance with claim 2 or claim 3 **characterised in that** the spherical screw nut (10) has engagement surfaces (14) which enable the placement of a spanner (15).

5. Securing system in accordance with one of the claims 1 to 4 **characterised in that** the shoulder (5) and a contact pressure surface (16) of the clamping screw (7) are executed as conical surfaces.

6. Securing system in accordance with claim 5 **characterised in that** the conical surfaces have a half cone angle 8° < α < 25°.

7. Securing system in accordance with claim 6 **characterised in that** the conical surfaces have a half cone angle α ≈ 20°.

8. Securing system in accordance with one of the claims 1 to 7 **characterised in that** the reception part (3) has a further mounting (17) for a connection rod (18).

9. Securing system in accordance with one of the claims 1 to 8 **characterised in that** the bone screw (1) has a collar (20) which projects outwardly over the inner diameter (19) of the shoulder (5).

10. Securing system in accordance with one of the claims 1 to 9 **characterised in that** the thread diameter (21) of the bone screw (1) which can be introduced into the bone is greater than the inner diameter (19) of the shoulder (5).

11. Securing system in accordance with one of the claims 2 to 4 **characterised in that** the spherical nut (10) has a neck (30) which forms a closed ring and which is supported outside the outer diameter of the screw-like extension (11) on a shoulder (20) of the bone screw (1).

12. Securing system in accordance with one of the claims 1 to 7 **characterised in that** the contact pressure surface (16) of the clamping screw (7) is provided with an inner thread (31) which is directed in the same sense as the outer thread (32) of the clamping screw (7) and has a similar pitch (33) in order that the inner thread (31) forms a groove on the spherical head part when the clamping screw (7) is tightened which improves the torque which can be transmitted to the spherical head part.

13. Securing screw in accordance with claim 12 **characterised in that** the inner thread (31) is executed with multiple turns in order to increase the number of the thread peaks acting on the head part (2).

## Revendications

1. Système de fixation pivotant entre une vis à os (1) avec une partie de tête sphérique (2) et une partie de réception (3) qui entoure à l'extrémité d'un perçage traversant (4) la partie de tête (2) avec un épaulement (5) au côté inférieur (6) de celle-ci et qui reçoit une vis de serrage (7) dans le perçage traversant (4), par laquelle la partie de tête (2) peut être appliquée par pression dans une position de pivotement sélectionnable (8) à l'épaulement (5), **caractérisé en ce que** la partie de tête sphérique (2) est réalisée comme partie séparée (9) qui peut être assemblée par vissage avec la vis à os pour mettre en place la partie de réception (3) après l'implant de la vis à os (1) et pour pouvoir l'assembler avec celle-ci.

2. Système de fixation selon la revendication 1, **caractérisé en ce que** la partie de tête sphérique (2) est réalisée comme écrou (10) qui peut être assemblé par vissage avec un prolongement hélicoïdal (11) de la vis à os (1).

3. Système de fixation selon la revendication 2, **caractérisé en ce que** l'écrou (10) est affaibli par au moins une fente de son filetage intérieur (13) vers l'extérieur pour fixer le filetage intérieur (13) lors de la compression de l'écrou sphérique (10) par une déformation des pas de vis sur le prolongement hélicoïdal (11).

4. Système de fixation selon la revendication 2 ou 3, **caractérisé en ce que** l'écrou sphérique (10) présente des faces d'engagement (14) qui permettent l'application d'un outil de vissage (15).

5. Système de fixation selon l'une des revendications 1 à 4, **caractérisé en ce que** l'épaulement (5) et une face d'application (16) de la vis de serrage (7) sont réalisés sous forme de faces coniques.

6. Système de fixation selon la revendication 5, **caractérisé en ce que** les faces coniques présente un demi angle conique 8° < α < 25°.

7. Système de fixation selon la revendication 6, **caractérisé en ce que** les faces coniques présentent un demi angle conique α ≈ 20°.

8. Système de fixation selon l'une des revendications 1 à 7, **caractérisé en ce que** la partie de réception (3) présente une fixation supplémentaire (17) pour une tige de liaison (18).

9. Système de fixation selon l'une des revendications 1 à 8, **caractérisé en ce que** la vis à os (1) présente un col (20) faisant saillie vers l'extérieur sur le diamètre intérieur (19) de l'épaulement (5).

10. Système de fixation selon l'une des revendications 1 à 9, **caractérisé en ce que** le diamètre de filetage (21) du filet de la vis à os (1) insérable dans l'os est plus grand que le diamètre intérieur (19) de l'épaulement (5).

11. Système de fixation selon l'une des revendications 2 à 4, **caractérisé en ce que** l'écrou sphérique (10) présente un col (30) qui forme un anneau fermé et qui prend appui à l'extérieur du diamètre extérieur du prolongement hélicoïdal (11) sur un épaulement (20) de la vis à os (1).

12. Système de fixation selon l'une des revendications 1 à 7, **caractérisé en ce que** la face d'application (16) de la vis de serrage (7) est pourvue d'un filetage intérieur (31) qui est orienté dans le même sens que le filetage extérieur (32) de la vis de serrage (7) et qui présente un même pas (33) pour que le filetage intérieur (31) lors du serrage de la vis de serrage (7) ménage sur la partie de tête sphérique (2) une rainure qui améliore le couple de rotation pouvant être transmis à la partie de tête sphérique.

13. Vis de fixation selon la revendication 12, **caractérisé en ce que** le filetage intérieur (31) est réalisé avec plusieurs pas pour augmenter le nombre des pointes de filetage attaquant à la partie de tête (2).
